Europäisches Patentamt

(19) European Patent Office

Office européen des brevets

(11) Publication number: **0 020 828**
**B1**

(12) **EUROPEAN PATENT SPECIFICATION**

(45) Date of publication of patent specification: 04.05.83

(21) Application number: 79301186.7

(22) Date of filing: 19.06.79

(51) Int. Cl.³: **C 07 C 43/275,**
**C 07 C 41/06, C 08 K 5/06**

(54) A method for the preparation of a plasticizer comprising di-tert.octyl-diphenyloxide and a plasticizer so obtained for use with resinous film-forming cellulosic derivatives.

(43) Date of publication of application:
07.01.81 Bulletin 81/1

(45) Publication of the grant of the patent:
04.05.83 Bulletin 83/18

(84) Designated Contracting States:
BE CH DE FR GB IT LU NL SE

(56) References cited:
DE - A - 1 810 179
FR - A - 2 126 600
FR - A - 2 254 322
GB - A - 1 350 893
US - A - 3 739 039

Chemical Abstracts, Vol. 46, (1952), 11682 a
Chemical Abstracts, Vol. 34, (1940), 1967

(73) Proprietor: AMERICAN POLYMERS, INC.
50 California Avenue
Paterson, New Jersey 07503 (US)

(72) Inventor: Klingel, Harry
274 Morris Turnpike
Summit, New Jersey 07901 (US)
Inventor: Ellison, Frank E
100 Franklin Street Building 4, Apt, B8
Morristown, New Jersey (US)

(74) Representative: Warden, John Christopher et al,
R.G.C. Jenkins & Co. 12-15, Fetter Lane
London EC4A 1PL (GB)

Courier Press, Leamington Spa, England.

## A method for the preparation of a plasticizer comprising di-tert. octyl-diphenyloxide and a plasticizer so obtained for use with resinous film-forming cellulosic derivatives

The present invention relates generally to certain alkylated diphenyl oxide derivatives useful as plasticizers for cellulosic derivatives, and particularly to a di-tertiary-octyl diphenyloxide possessing unique characteristics useful in the preparation of plasticized ethylcellulose compositions.

The preparation and general utility of alkylated diphenyloxide derivatives is known and has been described in U.S. Patent No. 2,170,809 to Coleman et al. The aforementioned disclosure broadly suggests the utility of this class of compounds as plasticizers, and provides a general synthesis involving the condensation of diphenyl-oxide or a lower alkyl derivative thereof with an alkylating agent such as an alkyl halide, olefin or alcohol, and an alkylating catalyst selected from the group of materials known as "Friedel-Crafts catalysts". The particular derivative of interest herein is prepared by the synthesis set forth in Example 7 of Coleman et al.

Further mention of the di-tertiary-octyl derivative appears in U.S. Patent No. 2,604,413, to Kropscott et al which relates to plasticized ethylcellulose compositions, wherein said derivative is reported as being prepared by an essentially Friedel-Crafts condensation.

Though the diphenyloxide derivatives taught by Coleman et al and Kropscott et al possess recognized utility as plasticizers, a need exists to further modify the plasticized ethyl-cellulose composition to provide an improvement in properties at a reduction in materials costs. This is particularly true in the instance under consideration herein, wherein the ethyl-cellulose material is desirably extruded to form a continuous thin film strip product. Ideally, the reduction of plasticizer content of the cellulosic resin without the corresponding diminution in processability would be desirable, as the presence of the plasticizer detracts from the tensile properties of the extruded strip product.

In accordance with the present invention, a plasticizer is disclosed which possesses a significantly reduced viscosity of 1000 to 2100 centistokes measured at 25°C wherein said plasticizer comprises di-tertiary-octyl-diphenyl-oxide. The present plasticizer is particularly useful in mixture with ethylcellulose, where favourable processability can be achieved together with the consequent retention of the full extent of the favourable properties which characterize the unplasticized resin.

The plasticizer of the present invention is prepared by a method comprising reacting diphenyloxide, di-isobutylene and a "Friedel-Crafts" catalyst, and washing the product of said reaction successively with aqueous solutions of hydrochloric acid and sodium carbonate, wherein intermediate and following said successive washing step, said product is subjected to at least one wash with hot water of temperature from 60°C to 100°C.

The plasticizer of the present invention exhibits a significantly reduced viscosity from that either achieved or expected with the comparable material disclosed in the art, as earlier set forth. By comparison, the prior art material has been measured to possess a viscosity ranging from 5000 to 7000 centistokes, measured at 25°C. This difference in viscosity enables the plasticizers of the present invention to serve in a comparable capacity at a reduced level in the composition. Moreover, the reduced viscosity enhances the processability of both the plasticizer and the plasticized resin.

The method of the present invention provides a readily reproducible technique for the preparation of the plasticizer, which confers greater economy of manufacture.

The plasticizer of the present invention may be employed in admixture with resinous cellulosic derivatives, specifically ethylcellulose, which are manufactured into continuous film or strip by an extrusion process. The resultant products possess favourable flexibility without evidence of a decrease in tensile properties from those of the resin alone.

A particular end use of the film or strip prepared, utilizing the plasticizer of this invention is the manufacture of inhibitor-strips installed in the detonating mechanisms of missile warheads and the like.

The plasticizer of this invention may be prepared by a process which comprises reacting diphenyloxide, di-isobutylene and a "Friedel-Crafts" catalyst and washing the product of said reaction a plurality of times with successive amounts of an aqueous solution of hydrochloric acid and sodium carbonate. Intermediate said successive washing steps, said product is subjected to at least one wash with hot water preferably while being maintained at a temperature of about 60°C.

Preferably said diphenyloxide is added first to a reaction vessel, said vessel is raised to about 60°C, said catalyst is added thereto in the ratio of 2.8 grams of catalyst for each mole of said diphenyloxide, and said di-isobutylene is subsequently added thereto.

Preferably said reaction between said diphenyloxide, said catalyst and said di-isobutylene extends for from 3 to 4 hours.

Preferably said reaction product is finally stripped at a temperature of 180°C subsequent to the completion of said washing steps.

The reaction conducted herein comprises a condensation involving diphenyloxide and di-isobutylene in the presence of an alkylation catalyst selected from those catalysts known to promote a "Friedel-Crafts" reaction. Di-isobutylene is commercially available as a mixture of about 80% of 2,4,4 trimethyl-1-pentene. "Friedel-Crafts" catalysts are well known in the art and comprise such materials as aluminum chloride, aluminum bromide, activated bleaching earths, ferric chloride and boron trifluoride. In accordance with the present invention, the preferred catalyst composition comprises anhydrous aluminum chloride.

In the preferred process, the above reaction is conducted at a temperature of about 60°C, in e.g. a glass-lined reaction vessel. The desired quantity of diphenyloxide is first added to the vessel and the temperature is raised to the 60°C level. After reaching the foregoing temperature, anhydrous aluminum chloride is added in the proportion of 2.8 grams of aluminum chloride for each mol of diphenyloxide. The resulting mixture is stirred until the aluminum chloride is completely dissolved. The di-isobutylene is finally added to this mixture with continuous agitation, over an extended period of from about 3 to $3\frac{1}{2}$ hours to facilitate the maintenance of the temperature of the mixture at or about 60°C. After the addition of di-isobutylene, the reaction is continued for an additional $\frac{1}{2}$ hour to assure completion.

The resulting reaction product is next washed with a 10% aqueous solution of hydrochloric acid of a quantity sufficient to remove the aluminum compounds from the product. During this wash, the product is vigorously agitated, as by stirring or the like, to enable the fullest possible interaction of the acid therewith, while the temperature is maintained at 60°C. After the agitation is stopped, the mixture is allowed to settle whereby the aqueous layer separates and is drained off.

Conventionally, the next operation in the preparation of the diphenyloxide comprises a wash with an aqueous solution of sodium carbonate for the purpose of neutralizing the product volume. In accordance with the present invention, however, it has been unexpectedly found that the viscosity of the final product may be significantly reduced by the employment of at least one hot water wash operation conducted intermediate and after said hydrochloric acid and said sodium carbonate rinses respectively. The washes of the present invention are conducted with hot water and rely on the same method of execution as is employed with said acid and said carbonate washes. Thus, the hot water is applied under agitation and at a temperature consistently maintained at about 60°C; and when the application of the water is completed, the product is allowed to settle in order that separation and drain-off of the aqueous layer may occur.

The hot water washes of the invention are conducted for a period of time varying with the volume of product involved. The temperature of the water generally ranges from about 60° to 100°C and, preferably, closely approximates the 60°C temperature level primarily employed in most all aspects of the present method. Though illustrated hereinbelow as employing singular hot water washes, the process of the present invention contemplates that multiple recurring washes may be used to more thoroughly flush the reaction product. When the above parameters are broadly observed, the resulting products are found to possess all of the claimed attributes of reduced viscosity and the like, desired in accordance with the present invention.

After the first series of hot water washes is conducted, the product is exposed to the aforementioned wash treatment comprising the 3% aqueous solution of sodium carbonate. As with the earlier washes, vigorous agitation and maintenance of temperature at or about 60°C is required. Completeness of this treatment may be checked by testing with Congo red indicator paper. Separation and drawing-off procedures employed herein are identical with the known practice.

After the completion of the above, the product is given an additional, final exposure to the aforementioned hot water, wash under the same conditions specified earlier. The resultant product is then vacuum stripped at a pressure of from 2 to 5 mm Hg (250 to 680 Pa), while being heated to 180°C to remove unreacted diphenyloxide, isobutylene and lower oligomers.

Once vacuum stripping is finished, the resultant product is transferred while hot to a container for storage, use or the like.

The invention will be better understood from a consideration of the following illustrative example.

Example

Three samples of a material determined as di-tertiary-octyl diphenyloxide were prepared for comparative testing. Sample A represented the material disclosed in U.S. Patent No. 2,604,413, in column 2, which was prepared with reference to Example 7 of U.S. Patent No. 2,170,809. Neither patent suggests the employment of the hot water washing steps of the present invention. Samples B and C were prepared by the preferred process above described.

## TABLE

Properties—theoretical and observed—for di-tertiary-octyl-diphenyloxide

|  | '413 Patent | A | B | C |
|---|---|---|---|---|
| Refractive index 20 C | 1.525 | 1.508 | 1.504 | 1.503 |
| Viscosity at 25°C (in centistokes) | 5,000-7,000 | 7400 | 1610 | 1232 |
| Colour | —— | Gardner 2 | Gardner 4 | Gardner 5 |
| Density 25°/4° | 0.944—.952 | 0.943 | 0.932 | 0.931 |

From the above table, it is apparent that a wide difference exists between Samples B and C representing the present invention and comparative Sample A, regarding the achieved viscosity of the product. Clearly, the samples prepared in accordance with the invention possess significantly reduced viscosities while retaining comparable levels of other critical properties such as colour, density and refractive index, which would suggest their utility in applications such as extrusion applications, where reduced viscosity is advantageous to processability.

## Claims

1. A method for the preparation of a plasticiser comprising di-tertiary octyl-diphenyloxide having a viscosity measured at 25°C of 1000 to 2100 centistokes, said method comprising reacting diphenyloxide, di-isobutylene and a "Friedel-Crafts" catalyst comprising aluminium chloride, washing the reaction product resulting therefrom successively with aqueous solutions of hydrochloric acid and sodium carbonate, characterised by the employment of at least one hot water wash operation conducted between and after said hydrochloric acid and said sodium carbonate washes respectively, wherein said hot water ranges in temperature from 60°C to 100°C.

2. A method according to claim 1 wherein said diphenyloxide is added first to a reaction vessel, the temperature of said vessel is raised to about 60°C, said catalyst is added thereto in the ratio of 2.8 grams of catalyst for each mole of said diphenyloxide, and said di-isobutylene is subsequently added thereto.

3. A method according to claim 1 or claim 2 wherein said reaction between said diphenyloxide, said catalyst and said di-isobutylene extends for from 3 to 4 hours.

4. A method according to any preceding claim wherein said reaction product is finally stripped at a temperature of 180°C subsequent to the completion of said washing steps.

5. A plasticizer for use with resinous film forming cellulosic derivatives which comprise di-tertiary-octyl-diphenyloxide having a viscosity measured at 25°C which ranges from about 1000 to about 2100 centistokes, said di-tertiary-octyl-diphenyloxide having been prepared by a method according to any preceding claim.

## Revendications

1. Procédé de préparation d'un plastifiant comprenant de l'oxyde de di-tert-octyl-diphényle ayant une viscosité mesurée à 25°C, de 1000 à 2100 centistokes, ce procédé consistant à faire réagir de l'oxyde de diphényle, du di-isobutylène et un catalyseur de "Friedel-Crafts" comprenant du chlorure d'aluminium, à laver le produit réactionnel obtenu successivement avec des solutions aqueuses d'acide chlorhydrique et de carbonate de sodium, caractérisé en ce qu'on utilise au moins une opération de lavage à l'eau chaude effectuée respectivement entre et après ces lavages à l'acide chlorhydrique et ces lavages au carbonate de sodium, la température de cette eau chaude étant comprise entre 60°C et 100°C.

2. Procédé selon la revendication 1, caractérisé en ce qu'on introduit tout d'abord cet oxyde de diphényle dans un récipient réactionnel, en ce qu'on porte la température de ce récipient à environ 60°C, en ce qu'on y introduit ce catalyseur à raison de 2,8 grammes de catalyseur par mole de cet oxyde de diphényle et en ce qu'on y introduit ensuite ce di-isobutylène.

3. Procédé selon les revendications 1 ou 2, caractérisé en ce que cette réaction entre cet oxyde de diphényle, ce catalyseur et ce di-isobutylène s'étend sur 3 à 4 heures.

4. Procédé selon l'une quelconque des revendications précédentes, caractérisé en ce que ce produit réactionnel est finalement purifié à une température de 180°C lorsque ces opérations de lavage sont achevées.

5. Plastifiant destiné à être utilisé avec des dérivés cellulosiques résineux transformables en pellicules, caractérisé en ce qu'il comprend de l'oxyde de di-tert-octyl-diphényle ayant une viscosité mesurée à 25°C d'environ 1000 à environ 2100 centistokes, cet oxyde de ditert-octyl-diphényle ayant été préparé par un procédé suivant l'une quelconque des revendications précédentes.

## Patentansprüche

1. Verfahren zur Herstellung eines Weichmachers aus Di-tert.octyldiphenyloxid, der eine Viskosität, gemessen bei 25°C, von 1000 bis 2100 Centistokes hat und in der Weise gewonnen wird, daß man Diphenyloxid, Diisobutylen und einen Friedel-Kraft-Katalysator aus Aluminiumchlorid miteinander zur Reaktion bringt und das dabei resultierende Reaktionsprodukt nacheinander mit wässriger Salzsäure und wässriger Natriumcarbonatlösung auswäscht, dadurch gekennzeichnet, daß man bei dem Auswaschvorgang sowohl zwischen als auch nach den Behandlungen mit Salzsäure bzw. Natriumcarbonatlösung wenigstens eine Auswaschbehandlung mit heißem Wasser vornimmt und dabei mit Heißwasser einer Temperatur im Bereich von 60 bis 100°C arbeitet.

2. Verfahren nach Anspruch 1, dadurch gekennzeichnet, daß zunächst das Diphenyloxid in einen Reaktionsbehälter eingegeben wird, dessen Temperatur auf etwa 60°C gebracht wird, dazu der Katalysator in einem Mengenverhältnis von 2,8 g an Katalysator je Mol an Diphenyloxid gegeben und anschließend das Diisobutylen zugefügt wird.

3. Verfahren nach einem der Ansprüche 1 oder 2, dadurch gekennzeichnet, daß man das Diphenyloxid, den Katalysator und das Diisobutylen während einer Zeitspanne von 3 bis 4 Stunden miteinander reagieren läßt.

4. Verfahren nach irgend einem der vorhergehende Ansprüche, dadurch gekennzeichnet, daß man nach dem vollständigen Auswaschen das Reaktionsprodukt zum Schluß einem Stripp-Vorgang bei einer Temperatur von 180°C unterzieht.

5. Weichmacher für harzartige, filmbildende Formmassen auf Basis von Cellulosederivaten, dadurch gekennzeichnet, daß er aus eine Viskosität, gemessen bei 25°C, im Bereich von etwa 1000 bis 2100 Centistokes aufweisendem Di-tert.octyldiphenyloxid, hergestellt nach dem in irgend einem der vorhergehenden Ansprüche beschriebenen Verfahren, besteht.